# EUROPEAN PATENT SPECIFICATION

(11) **EP 1 043 024 B1**
(45) Date of publication and mention of the grant of the patent: **08.06.2005**
(21) Application number: 00107099.4
(22) Date of filing: 06.04.2000
(51) Int. Cl.: A61K 38/21, A61K 47/48, A61P 35/00

(54) **Chronic myeloid leukemia (cml) therapy**
Chronische myeloide Leukämie Therapie
Thérapie de la leucémie myéloide chronique

(30) Priority: 08.04.1999 US 288369
(43) Date of publication of application: 11.10.2000
(60) Divisional application: 05002953.7
(73) Proprietor: SCHERING CORPORATION, Kenilworth, New Jersey 07033-0530 (US)
(72) Inventor: Rybak, Mary Ellen, Warren, NJ 07059 (US); Rose, Esther Helen, Westfield, NJ 07090 (US)
(74) Representative: UEXKÜLL & STOLBERG

(56) References cited:
- EP-A- 0 809 996
- WO-A-98/48840
- TALPAZ M ET AL: "Phase I study of polyethylene glycol (PEG) interferon alpha-2B (Intron-A) in CML patients." BLOOD, vol. 92, no. 10 SUPPL. 1 PART 1-2, 15 November 1998 (1998-11-15), page 251A XP000953118 ISSN: 0006-4971
- GUILHOT FRANCOIS ET AL: "Interferon alfa-2b combined with cytarabine versus interferon alone in chronic myelogenous leukemia." NEW ENGLAND JOURNAL OF MEDICINE, vol. 337, no. 4, 1997, pages 223-229, XP000953196 ISSN: 0028-4793
- TALPAZ M ET AL: "Phase I study of pegylated-interferon alpha-2a (PEGASYSTM) in patients with chronic myelogenous leukemia (CML)." BLOOD, vol. 94, no. 10 SUPPL. 1 PART 1, 15 November 1999 (1999-11-15), page 530a XP000953172 ISSN: 0006-4971

## Description

### BACKGROUND OF THE INVENTION

This invention relates to the use of pegylated interferon-alpha for the manufacture of a medicament for use in an improved therapy for treating patients having chronic myelocytic leukemia ("CML") by administering a therapeutically effective dose of pegylated interferon-alpha for a time sufficient to achieve at least a partial cytogenetic response.

Guilhot, F. et al. disclosed in N. Engl. J. Med., 1997, Volume 337, pages 223-229 that the combination of interferon alpha-2b and cytarabine increased the rate of major cytogenetic response and prolonged survival in patients in the chronic phase of CML. It must be noted that daily injections of interferon alpha-2b were required to achieve these results. In addition, interferon alpha-2b has many side effects that a substantial number of patients find unacceptable, and patient compliance with the daily injections of interferon alpha-2b has become a problemTalpaz, M. et al.. Blood 92: 251 a (1998) disclosed a Phase I study of pegylated interferon alpha in CML patients who were in a state of hematologic resistance to interferon alpha or were unable to tolerate interferon alpha. The study did not disclose treatment of patients not previously treated with interferon alpha. Allogenic bone marrow transplantation ("BMT") may be an alternative for CML patients with HLA-identical siblings. However, many patients are clearly too old or lack suitable donors and thus BMT is ruled out for most of these patients. (See the Editorial by Goldman, John M. in N. Engl. J. Med. 1997 volume 337, pages 270-271.) Accordingly, there is a need for an improved therapy for treating patients having CML.

### Summary of the Invention

The present invention provides the use of pegylated interferon-alpha for the manufacture of a medicament for use in a method of treating a patient having chronic myelogenous leukemia which comprises administering to such a patient a therapeutically effective dose of pegylated interferon alpha for a time period sufficient to effect at least a partial cytogenetic response.

The present invention also provides the use of pegylated interferon-alpha for the manufacture of a medicament for use in a method of treating a patient having chronic phase chronic myelogenous leukemia which comprises administering to said patient an effective amount of pegylated interferon-alpha once a week for a time period sufficient to effect at least a partial cytogenetic response.

The present invention further provides the use of pegylated interferon-alpha for the manufacture of a medicament for use in a method of a patient having chronic phase of chronic myelogenous leukemia which comprises administering to such a patient about 4.5 micrograms/kg to about 9.0 micrograms/kg of pegylated interferon alpha-2b once a week for a time period sufficient to effect at least a partial cytogenetic response.

### Detailed Description of the Invention

The present invention provides the use of pegylated interferon-alpha for the manufacture of a medicament for use in an improved method of treating patients with CML-especially those in the chronic phase of CML. The improved method provides a safer and more efficacious and tolerable treatment for CML by use of weekly injections of pegylated interferon alpha alone or in combination with chemotherapeutic agents such as cytarabine.The CML patients include those newly diagnosed with this disease The patients have never been treated with interferon alpha. Normally, hydroxyurea is given as needed to the CML patients before initiation of the method of the present invention to reduce the leukocyte count. Treatment with pegylated interferon alpha in accordance with the present invention will continue for a minimum of six months, and preferably for at least twelve months unless there is clinical evidence of disease progression, unacceptable toxicity or the patient requests that the therapy be discontinued.

When the pegylated interferon-alfa administered is a pegylated interferon alfa-2b, the therapeutically effective amount of pegylated interferon alfa-2b administered is in the range of about 4.5 to about 9.0 micrograms per kilogram of pegylated interferon alfa-2b administered once a week (QW), preferably about 4.5 to about 6.5 micrograms per kilogram of pegylated interferon alfa-2b administered once a week, more preferably about 5.5 to about 6.5 micrograms per kilogram of pegylated interferon alfa-2b administered once a week, and most preferably about 6.0 micrograms per kilogram of pegylated interferon alfa-2b administered once a week.

When the pegylated interferon-alfa administered is a pegylated interferon alfa-2a, the therapeutically effective amount of pegylated interferon alfa-2a administered is in the range of about 50 micrograms to about 500 micrograms once a week("QW"), preferably about 200 micrograms to about 250 micrograms QW.

The term "pegylated interferon alfa" as used herein means polyethylene glycol modified conjugates of interferon alfa, preferably interferon alfa-2a and -2b. The preferred polyethylene-glycol-interferon alfa -2b conjugate is PEG₁₂₀₀₀-interferon alfa 2b. The phrases "12,000 molecular weight polyethylene glycol conjugated interferon alpha" and "PEG₁₂₀₀₀-IFN alfa" as used herein mean conjugates such as are prepared according to the methods of International Application No. WO 95/13090 and containing urethane linkages between the interferon alfa-2a or -2b amino groups and polyethylene glycol having an average molecular weight of 12000.

The preferred PEG₁₂₀₀₀-interferon alfa-2b is prepared by attaching a PEG polymer to the epsilon amino group of a lysine residue in the IFN alfa-2b molecule. A single PEG₁₂₀₀₀ molecule is conjugated to free amino groups on an IFN alfa-2b molecule via a urethane linkage. This conjugate is characterized by the molecular weight of PEG₁₂₀₀₀ attached. The PEG₁₂₀₀₀-IFN alfa-2b conjugate is formulated as a lyophilized powder for injection. The objective of conjugation of IFN alfa with PEG is to improve the delivery of the protein by significantly prolonging its plasma half-life, and thereby provide protracted activity of IFN alfa.

The term " interferon-alfa " as used herein means the family of highly homologous species-specific proteins that inhibit viral replication and cellular proliferation and modulate immune response. Typical suitable interferon-alfas include, but are not limited to, recombinant interferon alfa-2b such as Intron-A interferon available from Schering Corporation, Kenilworth, N.J., recombinant interferon alfa-2a such as Roferon interferon available from Hoffmann-La Roche, Nutley, N.J., recombinant interferon alpha-2C such as Berofor alpha 2 interferon available from Boehringer Ingelheim Pharmaceutical, Inc., Ridgefield, CT., interferon alpha-n1, a purified blend of natural alfa interferons such as Sumiferon available from Sumitomo, Japan or as Wellferon interferon alpha-n1 (INS) available from the Glaxo-Wellcome Ltd., London, Great Britain, or a consensus alpha interferon such as those described in U.S. Patent Nos. 4,897,471 and 4,695,623 (especially Examples 7, 8 or 9 thereof) and the specific product available from Amgen, Inc., Newbury Park, CA, or interferon alfa-n3 a mixture of natural alfa interferons made by Interferon Sciences and available from the Purdue Frederick Co., Norwalk, CT., under the Alferon Tradename. The use of interferon alfa-2a or alpha-2b is preferred. Since interferon alpha-2b, among all interferons, has the broadest approval throughout the world for treating chronic hepatitis C infection, it is most preferred. The manufacture of interferon alpha-2b is described in U.S. Patent No. 4,530,901.

Other interferon alfa conjugates can be prepared by coupling an interferon alfa to a water-soluble polymer. A non-limiting list of such polymers include other polyalkylene oxide homopolymers such as polypropylene glycols, polyoxyethylenated polyols, copolymers thereof and block copolymers thereof. As an alternative to polyalkylene oxide-based polymers, effectively non-antigenic materials such as dextran, polyvinylpyrrolidones, polyacrylamides, polyvinyl alcohols, carbohydrate-based polymers and the like can be used. Such interferon alfa-polymer conjugates are described in U.S. Patent No. 4,766,106, U.S. Patent No. 4,917,888, European Patent Application No. 0 236 987, European Patent Application Nos. 0 510 356, 0 593 868 and 0 809 996( pegylated interferon alfa-2a) and International Publication No. WO 95/13090.

Pharmaceutical composition of pegylated interferon alfa-suitable for parenteral administration may be formulated with a suitable buffer, e.g., Tris-HCl, acetate or phosphate such as dibasic sodium phosphate/monobasic sodium phosphate buffer, and pharmaceutically acceptable excipients ( e.g., sucrose), carriers (e.g. human serum albumin), toxicity agents (e.g. NaCl), preservatives (e.g. thimerosol, cresol or benylalcohol), and surfactants( e.g. tween or polysorabates) in sterile water for injection. The pegylated interferon alfa-may be stored as lyophilized powders under a refrigeration at 2°-8°C. The reconstituted aqueous solutions are stable when stored between 2° and 8°C and used within 24 hours of reconstitution. See for example U.S. Patent Nos, 4,492,537; 5,762,923 and 5,766,582. The reconstituted aqueous solutionsmay also be stored in prefilled, multi-dose syringes such as those useful for delivery of drugs such as insulin. Typical suitable syringes include systems comprising a prefilled vial attached to a pen-type syringe such as the NOVOLET Novo Pen available from Novo Nordisk, as well as prefilled, pen-type syringes which allow easy self-injection by the user. Other syringe systems include a pen-type syringe comprising a glass cartridge containing a diluent and lyophilized pegylated interferon alfa powder in a separate compartment.

CML (chronic myelogenous leukemia) is a clonal myeloproliferative disorder which is a neoplastic proliferation of the pluripotential stem cell. In CML, the leukemic cells retain some ability to differentiate, hence, at the time CML is diagnosed, the white cell count in CML may range from 10,000 to >200,000 cells/mm³ with 90% of the cells in the granulocyte series. Hematocrit, hemoglobin and platelet counts are usually normal although the platelet count and number of basophils may be increased. CML was the first cancer to be associated with a specific cytogenetic abnormality, the Philadelphia chromosome (Ph¹), a reciprocal translocation involving chromosomes 22 and 9. A segment of the long arm of chromosome 9 which contains the c-abl oncogene is translocated to the q11 position on chromosome 22 within a specified segment designated as the breakpoint cluster region (bcr). This results in a new gene, bcr/abl, on chromosome 22 within an associated abnormal messenger RNA, which can be detected by RT-PCR (reverse transcriptase polymerase chain reaction), and an abnormal protein product. A bcr/abl gene rearrangement is the major pathogenic mechanism underlying the development of CML.

The term "cytogenic response" as used herein means a reduction or elimination of Philadelphia chromosone-positive cells ("Ph¹ + cells") in the bone marrow. A complete cytogenetic response means there are no Ph¹ + cells; a major cytogenetic response means there are about 1 to about 34% of such cells i.e. < about 35% Ph¹ + cells. Minor response means about 35 to about 90% of such cells and treatment failure about 91 to about 100% of such cells i.e., >about 90% Ph¹ + cells). Clinicans have suggestd that achievement of a major cytogenic response i.e. <about 35% of Ph + cells in the bone marrow after 1 year of CML therapy is predictive of long-term survival.

The term "patients having chronic myelogenous leukemia or CML" as used herein means any patient having CML and includes treatment-naive patients as well as treatment-experienced patients as well as patients in the chronic phase of CML.

The term "treatment-naive patients" as used herein means patients with CML-including newly-diagnosed CML patients- who have never been treated with any chemotherapeutic drugs, including but not limited to, e.g., busulfan("BU"), hydroxyurea("HU"), Homoharringtonine ("HHT"), cytarabine("Ara-C"), Idadubicin("I"), Etoposide("E") or chemotherapeutic drug combinations, e.g., I + Ara-C + E, i.e.,"ICE" as well as any interferon, including but not limited to interferon alfa, or pegylated interferon alfa.

The term "treatment-experienced patients as used herein means those patients who have initiated some form of chemotherapeutic drug therapy including, but not limited to chemotherapeutic drug, e.g., busulfan("BU"), hydroxyurea("HU"), Homoharringtonine ("HHT"), cytarabine("Ara-C"), Idadubicin("I"), Etoposide("E") or chemotherapeutic drug combinations, e.g., "ICE".

The term "hematologic response" as used herein means an improvement in the WBC, and platelets.

A complete hematologic response means a WBC of less than 10,000 per microliter and a platelet count of less than 450,000 per microliter and normal differential in peripheral blood, and no.palpable spleen.

A partial hematologic response means a WBC of fewer than about 20,000 per microliter, or at least about a 50% reduction in the WBC baseline (measured pretreatment).

In a preferred embodiment of the present invention, hydroxyurea is administered to CML patients prior to initiation of pegylated interferon alfa, and preferably about two weeks up to about three months prior to initiation of pegylated interferon alfa.

Pegylated interferon-alfa formulations are not effective when administered orally, so the preferred method of administering the pegylated interferon-alfa is parenterally, preferably by subcutaneous, IV, or IM, injection. Of course, other types of administration of both medicaments, as they become available are contemplated, such as by nasal spray, transdermally, by suppository, by sustained release dosage form, and by pulmonary inhalation. Any form of administration will work so long as the proper dosages are delivered without destroying the active ingredient.

The following Clinical Study Design may be used to treat CML patients in accordance with the method of the present invention. Many modifications of this Clinical Study Design protocol will be obvious to the skilled clinician, and the following Study Design should not be interpreted as limiting the scope of the method of this invention which is defined by the claims listed hereinafter.

### Clinical Study Design

In a preferred embodiment of the CML treatment method of the present invention, subjects newly diagnosed with CML may be administered hydroxyurea for a maximum of up to3 months in order to control WBC counts. For subjects who present with WBC 50,000µl, treatment with hdroxyurea is not necessary. "Date of diagnosis" will be considered to be the date that the subject is confirmed to be Ph¹+ by cytogenetic testing (performed at any qualified laboratory using RT-PCR). Radomization into this study should take place within 3 months of initial diagnosis, and after the WBC 50,000µl. Subjects who do not achieve a WBC 50,000µl or who have progressive splenomegaly after pretreatment with hydroxyurea will not be randomized to receive study drug. Subjects who do achieve a WBC 50,000µl without progressive splenomegalyafter up to about 3 months of hydroxyurea therapy therapy will be randomized to one of two treatment groups A & B as follows: The subjects will receive pegylated interferon alfa 2b, i.e., PEG₁₂₀₀₀-interferon alfa 2b at doses of 6.0 micrograms per kilogram by subcutaneous injection once a week (**Group B**) or interferon alfa-2b at doses of 5 million international units per square meter of body surface area per day ("5 MIU/m²/day") (**Group A**).
**Group A: INTRON® A(interferon alfa-2b, recombiant)**
   5 MIU/m² daily by SC injection
**Group B: PEG Intron (**PEG₁₂₀₀₀**-interferon alfa-2b recombiant)**
   6.0 µg/kg weekly by SC injection

### Duration of Study and Visit Schedule

The duration of this study is based upon achieveing a therapeutic response, and will be determined for each subject individually.

Treatment with either PEG Intron or INTRON® A will continue for a minimum of 6 months unless there is evidence of disease progression, unacceptable toxicity, or the subject requests that therapy be discontinued. Hematological response will be assessed at 3, 6, 9 and 12 months and cytogenetic response will be evaluated at 6 and 12 months during the first year of study treatment. Subjects achieveing a complete hematologic response at 3 months should have the cytogenetic response evaluated at 3 months as well. Population pharmacokinetics will be conducted at various timepoints throughout the study. In addition, quality of life and overall survival data will be collected. Subjects who achieve a complete hematologic response by 6 months will continue treatment for another 6 months.

At the end of six months of treatment, the subject's hematologic response will be evaluated to determine if the subject has achieved a complete hematologic response. Subjects achieving a complete hematologic response can continue treatment.

Criteria for Complete Hematologic Response:
- WBC <10,000/µl
- Platelets <450,000/µl
- Normal differential in peripheral blood (PB)
- No palpable spleen

Subjects who fail to achieve a complete hematologic response after 6 months of treatment will be considered treatment failures. Further treatment for this group will be at the discretion of the treating physician. Subjects may continue to receive their assigned study medication for an additional 6 months on this protocol. The addition of Ara-C will be permitted for subjects already determined to be treatment failures. These subjects will continue the scheduled study evaluations, including the 12-month cytogenetic assessment.
- After one year of treatment, subject who have achieved at least a partial cytogenetic response (90% Ph¹+) may continue study treatment until disease progression. Subjects who fail to achieve a minor cytogenetic response after 1 year of treatment will be considered to be treatment failure and study treatment will be discontinued. After 2 years of treatment, subjects who have achieved a major cytogenetic response (35% Ph¹+ cells) may continue study treatment until disease progression. Subjects who have not achieved a major cytogenetic response will be discontinued from the study. All subjects will be followed by survival, regardless of when they go off study.

After 12 months of treatment, subjects with a minor cytogenetic response (i.e., 90% Ph¹+ cells) will be eligible to continue treatment for an additional 12 months. After 2 years of treatment, subjects with a Partial or Complete Cytogenetic Response (i.e., <35% Ph¹+ cells) may continue treatment until disease progression.

The goal of the improved CML therapy of the present invention is to achieve at least a partial cytogenetic response
(< 90% Ph¹ + cells) after 6 to 12 months of treatment and preferably to achieve a Partial or Complete Cytogenetic Response (< 35% Ph¹ + cells) after 12 to 24 months of treatment.

The following clinical protocol may be used to administer the CML therapy of the present invention:
The study population will include male and female patients with newly diagnosed CML i who are treatment naive and will be included if they meet the following inclusion and exclusion criteria:

### Subject Inclusion Criteria

a) Subjects must have chronic phase CML diagnosed within 3 months prior to study enrollment. Date of diagnosis is the date of first documentation of the presence of the Philadelphia chromosome (Ph¹+) as confirmed by cytogenetic testing, performed by a central laboratory.
b) Subjects must have chronic phase CML that is positive for Ph¹+ cells as confirmed by cytogenetic studies, performed by a central laboratory.
c) Subjects must meet or exceed the following hematologic criteria:
   - Platelet count ≥50,000/µl
   - Hemoglobin ≥9.0 g/dL
   - WBC count ≥2000/µl bur 50,00/µl
d) Subjects must have adequate hepatic and renal function as defined by the following parameters, obtained within 14 days prior to initiation of study treatment:
   - SGOT and SGPT <2 times upper limit of laboratory normal (ULN)
   - Plasma bilirubin <2 times ULN
   - Plasma creatinine <2.0 mg/dL
e) Subjects must be fully recovered from any prior major surgery and must be at least 4 weeks postperative.
f) Subjects must be between 18-70 years old.
g) Subjects must have ECOG Performance Status of 0-2
h) Subjects must sign a written, voluntary informed consent before study entry, be willing to participate in this study and be willing to complete all follow-up assessments.

### Subject Exclusion Criteria

a) Subjects with accelerated or blastic phase CML as defined by the following criteria.
   **Criteria for accelerated phase CML (any of the following):**
   Peripheral blood myeloblasts 15%
   Peripheral blood basophils 20%
   Peripheral blood myeloblasts plus proyelocytes 30%
   Platelets <100,000/µl unrelated to therapy
   **Criteria for blast CML:**
   30% myeloblasts in peripheral blood or bone marrow
b) Subjects who are candidates for and are planning to received allogeneic, syngeneic, or autologous bone marrow transplantation (BMT) within the next 12 months.
c) Subjects who have received prior treatment for their CML, except for hydroxyurea.
d) Subjects who have severe cardiovascular disease, i.e., arrhythmias requiring chronic treatment, congestive heart failure (NYHA Class III or IV), symptomatic ischemic heart disease.
e) Subjects with a history of a neuropsychiatric disorder requiring hospitalization.
f) Subjects with thyroid dysfunction not responsive to therapy.
g) Subjects with uncontrolled diabetes mellitus.
h) Subjects who have a history of seropositivity for HIV.
i) Subjects with active and/or uncontrolled infection, including active hepatitis.
j) Subjects with a medical condition requiring chronic systemic corticosteroids.
k) Subjects with a history of prior malignancies within the last 5 years, except for surgically cured non-melanoma skin cancer, or cervical carcinoma in situ.
l) Subjects who have reaceived any experimental therapy within 30 days prior to enrollment in this study.
m) Subjects who are known to be actively abusing alcohol or drugs.
n) Subjects who are pregnant, nursing, or of reproductive potential and who are not practicing an effective means of contraception.

### Subject Discontinuation Criteria

It is the right and duty of the investigator to inerrupt the treatment of any subject whose health or well being may be threatened by continuation in this study.

Subjects may be discontinued prior to completion of the study for any of the following reasons:
a) Experiences the onset of accelerated or blast phase CML.
b) The WBC rises over 100,000/µl after 3 months of treatment with either INTRON® A or PEG Intron despite treatment with hydroxyurea. Treatment with hydroxyurea is prohibited after 3 months on study.
c) Has a clinically significant adverse event as determined by the Principal Investigator.
d) Does not achieve the desired therapeutic responses at 6 and 12 months.
e) Requests to be withdrawn from the study.
f) Fails to comply with the requirements for study evaluaitonslvisits.
g) Circumstances develop which prevent study evaluations/visits.
h) Develops other conditions for which, in the investigator's opinion, it is in the subject's best interest to be withdrawn from the study.
i) Develops severe depression or any other psychiatric disorder requiring hospitalization.
j) Experiences a serious allergic rfesponse to the study drug manifested by angioedema, bronchoconstriction, or anaphylaxis.
k) Experiences recurrent toxicities despite dose reductions.
l) Receives treatment with a prohibited medication.

### Analysis of Primary and Secondary Endpoints

The primary efficacy endpoint will be the cytogenetic response at 12 months. The primary analysis will be the comparison of treatment groups with respect to the proportion of subjects with major cytogenetic response at 12 months using the Cochran Mantel-Haenszel test adjusting for strata. Odds ratio and 95% confidence intervals for the odds ratio will be summarized. The analysis will be performed on an intent-to-treat basis. The primary analysis will be based on cytogenetic response where responders are those subjects who were not treatment failures and who had major cytogenetic response (<35% Ph¹+ cells) at 12 months. In this analysis, subjects who were treatment failures at 6 months will be considered cytogenetic non-responders. In a secondary analysis of cytogenetic response, subjects will be analyzed according to their cytogenetic response at 12 months, regardless of whether they were treatment failures or not.

The secondary endpoints of the study will be cytogenic response at 6 months, hematologic response at 3, 6 and 12 months, and overall survival. Cytogenetic response at 6 months and hematologic response at 3, 6 and 12 months will be analyzed using the Cochran Mantel-Haenszel test. Overall survival will be analyzed using the log-rank statistic. Kaplan-Meier estimates of the survival curves will be provided. Hazard ratio and 95% confidence interval for the hazard ratio will be obtained using Cox's proportional hazards model.

## Claims

1. Use of pegylated interferon alpha for the manufacture of a medicament for treating chronic myelogenous leukemia in a patient who has never been treated with interferon alpha.

2. The use according to claim 1, wherein the pegylated interferon alpha is pegylated interferon alpha-2a or pegylated interferon alpha-2b.

3. The use according to claim 1 or 2, wherein the patient is a treatment-naive patient.

4. The use according to claim 3, wherein the treatment-naive patient is one having newly diagnosed chronic phase chronic myelogenous leukemia.

5. The use according to claim 1 or 2, wherein the patient is a treatment-experienced patient.

6. The use according to claims 1-5, wherein the patient has chronic phase chronic myelogenous leukemia and the pegylated interferon alpha is formulated for delivery once a week for a time period sufficient to effect at least a partial cytogenetic response.

7. The use of claims 1-6, wherein the medicament is formulated for delivery for a time period of at least 6 months.

8. The use of claim 7, wherein the medicament is formulated for delivery for a time period of at least 12 months.

9. The use of claims 1-8, wherein the medicament is sufficient to effect at least a partial cytogenetic response in the patient.

10. The use according to claims 2-9, wherein the pegylated interferon alpha is pegylated interferon alpha-2b and the amount of pegylated interferon alpha-2b is in the range of about 4.5 micrograms/kg to about 9.5 micrograms/kg administered once a week.

11. The use according to claim 10, wherein the amount of pegylated interferon alpha-2b is in the range of about 4.5 micrograms/kg to about 6.5 micrograms/kg.

12. The use according to claim 11, wherein the amount of pegylated interferon alpha-2b is in the range of about 5.5 micrograms/kg to about 6.5 micrograms/kg.

13. The use according to claims 2-9, wherein the pegylated interferon alpha is pegylated interferon alpha-2a and the amount of pegylated interferon alpha-2a is in the range of about 50 to about 500 micrograms administered once a week.

14. The use according to claim 13, wherein the amount of pegylated interferon alpha-2a is in the range of about 200 to about 250 micrograms.

15. The use of claims 1-14, wherein the medicament further comprises cytarabine.

16. The use according to claims 1-15, wherein the pegylated interferon alpha comprises polyethylene glycol having an average molecular weight of 12,000.

17. The use according to claim 16, wherein polyethylene glycol is conjugated to the interferon alpha via a urethane linkage.

18. The use according to claim 1-17, wherein the pegylated interferon alpha is formulated as a lyophilized powder suitable for injection.

## Patentansprüche

1. Verwendung von pegyliertem Interferon alpha für die Herstellung eines Arzneimittels zur Behandlung chronischer Myeloischer Leukämie eines Patienten, der zuvor noch nicht mit Interferon alpha behandelt wurde.

2. Verwendung gemäß Anspruch 1, bei der das pegylierte Interferon alpha pegyliertes Interferon alpha-2a oder pegyliertes Interferon alpha-2b ist.

3. Verwendung gemäß Anspruch 1 oder 2, bei der der Patient ein unbehandelter Patient ist.

4. Verwendung gemäß Anspruch 3, bei der der behandelte Patient ein solcher ist, bei dem die chronische Phase der chronischen Myeloischen Leukämie neu diagnostiziert wurde.

5. Verwendung gemäß Anspruch 1 oder 2, bei der der Patient ein vorbehandelter Patient ist.

6. Verwendung gemäß einem der Ansprüche 1 bis 5, bei der sich der Patient in der chronischen Phase einer chronischen Myeloischen Leukämie befindet und das pegylierte Interferon alpha für die einmal wöchentliche Verabreichung für einen Zeitraum formuliert wurde, der ausreicht, um mindestens eine teilweise cytogenetische Antwort zu erzeugen.

7. Verwendung gemäß einem der Ansprüche 1 bis 6, bei der das Medikament für Verabreichung über einen Zeitraum von mindestens 6 Monaten formuliert ist.

8. Verwendung des Anspruchs 7, bei der das Medikament für eine Verabreichung über einen Zeitraum von mindestens 12 Monaten formuliert ist.

9. Verwendung gemäß einem der Ansprüche 1 bis 8, bei der das Medikament ausreicht, um wenigstens eine teilweise cytogenetische Reaktion in dem Patienten zu erzeugen.

10. Verwendung gemäß einem der Ansprüche 2 bis 9, bei der das pegylierte Interferon alpha pegyliertes Interferon alpha-2b und die Menge des pegylierten Interferon alpha-2b im Bereich von etwa 4,5 Mikrogramm/kg bis etwa 9,5 Mikrogramm/kg liegt, die einmal wöchentlich verabreicht werden.

11. Verwendung gemäß Anspruch 10, bei der die Menge des pegylierten Interferon alpha-2b im Bereich von etwa 4,5 Mikrogramm/kg bis etwa 6,5 Mikrogramm/kg liegt.

12. Verwendung gemäß Anspruch 11, bei der die Menge des pegylierten Interferon alpha-2b im Bereich von etwa 5,5 Mikrogramm/kg bis etwa 6,5 Mikrogramm/kg liegt.

13. Verwendung gemäß einem der Ansprüche 2 bis 12, bei der das pegylierte Interferon alpha pegyliertes Interferon alpha-2a ist und die Menge des pegylierten Interferon alpha-2a im Bereich von etwa 50 bis etwa 500 Mikrogramm liegt, die ein einmal wöchentlich verabreicht werden.

14. Verwendung gemäß Anspruch 13, bei der die Menge des pegylierten Interferon alpha-2a im Bereich von 200 bis 250 Mikrogramm liegt.

15. Verwendung gemäß einem der Ansprüche 1 bis 14, bei der das Arzneimittel ferner Cytarabin umfaßt.

16. Verwendung gemäß einem der Ansprüche 1 bis 15, bei der das pegylierte Interferon alpha Polyethylenglycol mit einem durchschnittlichen Molekulargewicht von 12.000 umfaßt.

17. Verwendung gemäß Anspruch 16, bei der das Polyethylenglycol an das Interferon alpha mittels einer Urethanbindung gebunden wurde.

18. Verwendung gemäß einem der Ansprüche 1 bis 17, bei dem das pegylierte Interferon alpha als lyophilisiertes Pulver formuliert wurde, das für die Injektion geeignet ist.

## Revendications

1. Utilisation d'interféron alpha pégylé pour la préparation d'un médicament destiné au traitement de la leucémie myélogène chronique chez un patient qui n'a jamais été traité avec de l'interféron alpha.

2. Utilisation selon la revendication 1, dans laquelle l'interféron alpha pégylé est un interféron alpha-2a pégylé ou un interféron alpha-2b pégylé.

3. Utilisation selon la revendication 1 ou 2, dans laquelle le patient est un patient vierge de traitement.

4. Utilisation selon la revendication 3, dans laquelle le patient vierge de traitement est un patient ayant une leucémie myélogène chronique, en phase chronique, nouvellement diagnostiquée.

5. Utilisation selon la revendication 1 ou 2, dans laquelle le patient est un patient ayant fait l'expérience d'un traitement.

6. Utilisation selon l'une quelconque des revendications 1 à 5, dans laquelle le patient est un patient ayant une leucémie myélogène chronique, en phase chronique, et l'interféron alpha pégylé est mis sous une forme prévue pour une administration une fois par semaine, pendant une durée suffisante pour obtenir au moins une réponse cytogénétique partielle.

7. Utilisation selon l'une quelconque des revendications 1 à 6, dans laquelle le médicament est mis sous une forme prévue pour une administration pendant une durée d'au moins 6 mois.

8. Utilisation selon la revendication 7, dans laquelle le médicament est mis sous une forme prévue pour une administration pendant une durée d'au moins 12 mois .

9. Utilisation selon l'une quelconque des revendications 1 à 8, dans laquelle le médicament est suffisant pour produire chez le patient au moins une réponse cytogénétique partielle.

10. Utilisation selon l'une quelconque des revendications 2 à 9, dans laquelle l'interféron alpha pégylé est un interféron alpha-2b pégylé, et la dose d'interféron alpha-2b pégylé est comprise dans l'intervalle allant d'environ 4,5 µg/kg à environ 9,5 µg/kg, cette dose étant administrée une fois par semaine.

11. Utilisation selon la revendication 10, dans laquelle la dose d'interféron alpha-2b pégylé est comprise dans l'intervalle allant d'environ 4,5 µg/kg à environ 6,5 µg/kg.

12. Utilisation selon la revendication 11, dans laquelle la dose d'interféron alpha-2b pégylé est comprise dans l'intervalle allant d'environ 5,5 µg/kg à environ 6,5 µg/kg.

13. Utilisation selon l'une quelconque des revendications 2 à 9, dans laquelle l'interféron alpha pégylé est un interféron alpha-2a pégylé, et la dose d'interféron alpha-2a pégylé est comprise dans l'intervalle allant d'environ 50 à environ 500 µg, cette dose étant administrée une fois par semaine.

14. Utilisation selon la revendication 13, dans laquelle la dose d'interféron alpha-2a pégylé est comprise dans l'intervalle allant d'environ 200 à environ 250 µg.

15. Utilisation selon l'une quelconque des revendications 1 à 14, dans laquelle le médicament contient en outre de la cytarabine.

16. Utilisation selon l'une quelconque des revendications 1 à 15, dans laquelle l'interféron alpha pégylé est un produit comprenant du polyéthylèneglycol ayant une masse moléculaire moyenne de 12 000.

17. Utilisation selon la revendication 16, dans laquelle le polyéthylèneglycol est uni à l'interféron alpha par l'intermédiaire d'une liaison uréthane.

18. Utilisation selon l'une quelconque des revendications 1 à 17, dans laquelle l'interféron alpha pégylé est mis sous la forme d'une poudre lyophilisée, appropriée pour une injection.
